# EUROPEAN PATENT APPLICATION

(11) **EP 2 196 458 A1**
(43) Date of publication of application: **16.06.2010**
(21) Application number: 08382074.6
(22) Date of filing: 10.12.2008
(51) Int. Cl.: C07D 231/54

(54) **Process for obtaining enantiomerically enriched pyrazole derivatives**

(71) Applicant: Laboratorios Del. Dr. Esteve, S.A., 08041 Barcelona (ES)
(72) Inventor: Corbera, Arjona, Jordi, 08225 Terrassa (ES)
(74) Representative: ABG Patentes, S.L.

(57) **Abstract**

The present invention is directed to a process for the enantioselective hydrolysis of an ester of formula (I), which comprises reacting a mixture of C4-enantiomers of esters of formula (I), or salts thereof, with an esterase enzyme in the presence of a solvent. The invention is further directed to the transformation of said esters of formula (I) into enantiomerically enriched pyrazoles.

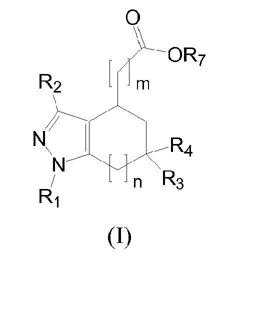

## Description

### FIELD OF THE INVENTION

The present invention relates to a process for the synthesis of enantiomerically enriched intermediate compounds, wherein the key step is an enantioselective hydrolysis of an ester. The invention further relates to the transformation of the resulting enantiomerically enriched esters into known pyrazole derivatives useful for the treatment of psychosis or pain.

### BACKGROUND OF THE INVENTION

WO 2006/021463 A1 discloses pyrazole derivatives, their synthesis and pharmaceutical compositions comprising them, for the treatment and prophylaxis of psychosis or pain. Concretely, WO 2006/021463 A1 disclosed for the first time a family of pyrazole derivatives with inhibitory activity over the sigma receptor. Where said pyrazole derivatives comprised a chiral centre in carbon 4 (e.g. compounds of formula (Ia)), WO 2006/021463 A1 disclosed a synthetic process for their preparation involving a racemic synthesis, the last step of which comprised the resolution through chiral semi-preparative HPLC.

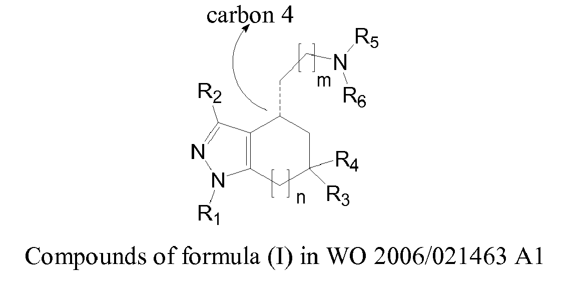

Since both of the enantiomers are active inhibitors of the sigma receptor, it would be desirable to provide an alternative synthetic procedure giving access to both enantiomers in optically pure form. In addition, it would also be desirable to propose a process that will offer higher yields and that will turn out intermediates and end products in high enantiomeric excess from an early stage in the synthesis. This last feature would facilitate the industrial scale-up of each of the enantiomers.

Rudolph J et al. J. Med. Chem., 2007, 50, 984-1000 and Wickens P et al. Bioorg. Med. Chem. Lett., 2007, 17, 4369-4373 discloses the enzymatic hydrolysis of an indan-1-yl-acetic acid ethyl ester with Amano Lipase PS into the corresponding (S)-indane acetic acid and the corresponding (*R*)-indane acetic acid ethyl ester.

Bueno AB et al. Bioorg. Med. Chem. Lett., 2007, 17, 3344-3348 discloses the resolution of a racemic mixture of a substituted (2,3-dihydro-benzofuran-3-yl)-acetic acid ethyl ester using Amano lipase, obtaining the *dextro* ester isomer and the *levo* acid isomer in 45% yield and 95% ee for each enantiomer.

Truppo MD et al., Org. Process Res. Dev., 2006, 10, 592-598 discloses the Amano AK-AF lipase catalysed hydrolysis of a racemic indole-ethyl ester into the (*S*)-acid enantiomer and the (*R*)-ester in 95% ee.

Bellur E et al., Tetrahedron 2006, 62, 7132-7139 discloses the recombinant esterase Est 56 kinetic resolution of (tetrahydrofuran-2-yl)acetates.

Roberts NJ et al., Green Chem., 2004, 6, 475-482 discloses the Candida antarctica lipase B (Novozyme 435 lipase) catalysed resolution of 2,3,4,5-tetrahydro-4-methyl-3-oxo-1*H*-1,4-benzodiazepine-2-acetic acid methyl ester (SB-235349) into the (*S*)-acid, and the corresponding (*R*)-ester.

Kirschner A et al., Tetrahedron: Asymmetr. 2004, 15, 2871-2874 discloses among others, the Amano PS lipase-catalyzed kinetic resolution of racemic α-hydroxy butenolides into the corresponding *dextro* ester and *levo* acid enantiomers.

Atkins RJ et al., Org. Process Res. Dev., 2003, 7, 663-675 discloses lipase-catalyzed resolution of racemic SB-235349 into the corresponding (*S*)-acid and (*R*)-ester isomers.

### BRIEF DESCRIPTION OF THE FIGURES

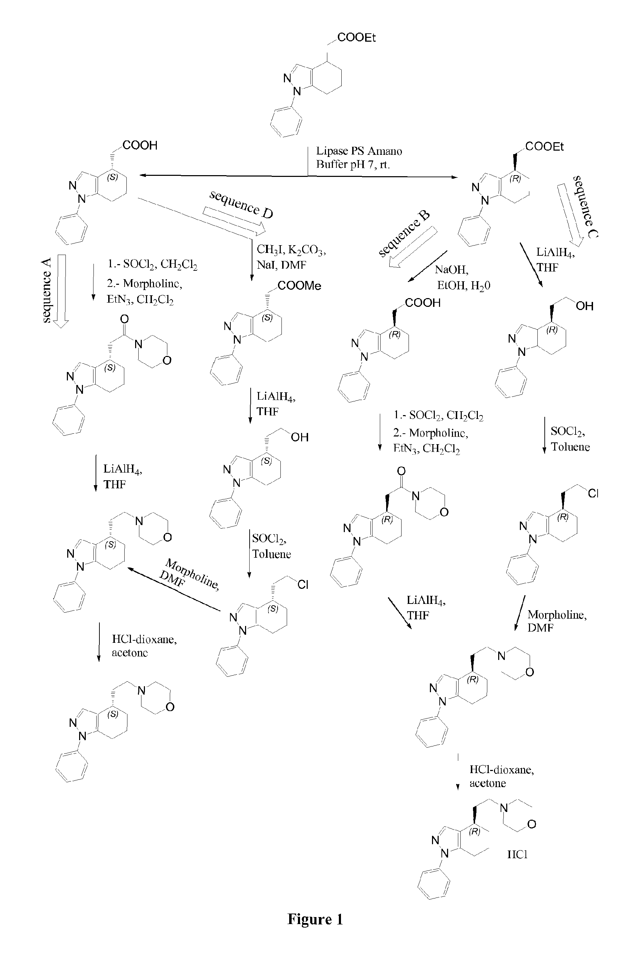

**Figure 1** shows a schematic view of the synthesis of compounds (+)-(*R*)-4,5,6,7-tetrahydro-4-[2-(morpholin-4-yl)ethyl]-1-phenyl-1H-**indazole and** (-)-(*S*)-4,5,6,7-tetrahydro-4-[2-(morpholin-4-yl)ethyl]-1-phenyl-1H-indazole, and the hydrochloride salts thereof.

### SUMMARY OF THE INVENTION

In their ongoing efforts to provide an alternative route to enantiomerically enriched, preferably enantiomerically pure, pyrazole derivatives disclosed in WO 2006/021463 A1, the inventors have surprisingly found that enzymatically catalyzed hydrolysis of the esters of formula (I) defined below opens an alternative route to both enantiomers of the mentioned pyrazole derivatives.

Thus, according to a first aspect, the present invention is directed to a process for the enantioselective hydrolysis of an ester of formula (I), which comprises reacting a mixture of C4-enantiomers of esters of formula (I), or salts thereof, with an esterase enzyme in the presence of a solvent 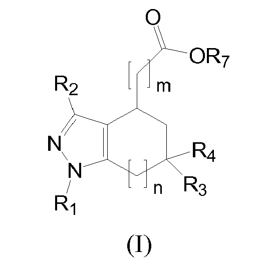 wherein
R₁ is selected from the group formed by hydrogen, substituted or unsubstituted alkyl, substituted or unsubstituted cycloalkyl, substituted or unsubstituted heterocyclyl, substituted or unsubstituted aryl, substituted or unsubstituted arylalkyl, and substituted or unsubstituted heterocyclylalkyl;
R₂ is selected from the group formed by hydrogen, substituted or unsubstituted alkyl, substituted or unsubstituted cycloalkyl, substituted or unsubstituted alkoxy, substituted or unsubstituted aryl, substituted or unsubstituted heterocyclyl, substituted or unsubstituted arylalkyl, and substituted or unsubstituted heterocyclylalkyl;
R₃ and R₄ are independently selected from the group formed by hydrogen, substituted or unsubstituted alkyl, substituted or unsubstituted cycloalkyl, substituted or unsubstituted heterocyclyl, substituted or unsubstituted aryl, substituted or unsubstituted arylalkyl and substituted or unsubstituted heterocyclylalkyl or, together, R₃ and R₄ form a 3 to 6 substituted or unsubstituted member ring;
R₇ is selected from the group formed by substituted or unsubstituted alkyl and substituted or unsubstituted cycloalkyl;
n is selected from 0, 1 and 2; and
m is selected from 0, 1, 2, 3 and 4.

Further, a second aspect of the invention is directed to process for the preparation of an enantiomerically enriched ester of formula (I) , or salts thereof, an enantiomerically enriched acid of formula (II), or salts thereof, or a mixture of an enantiomerically enriched ester of formula (I) and an enantiomerically enriched acid of formula (II) 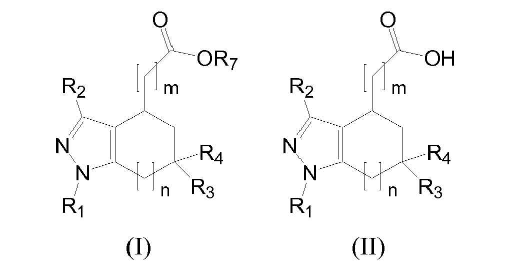 wherein R₁, R₂, R₃, R₄, R₇, n and m are as defined above;
comprising the step of reacting a mixture of C4-enantiomers of the ester of formula (I), or salts thereof, with an esterase enzyme in the presence of a solvent.

The process of the present invention provides for he first time enantiomerically enriched esters of formula (I) and/or acids of formula (II), used in their racemic form in WO 2006/021463 A1.

Accordingly, a further aspect of the present invention is a process for the synthesis of an enantiomerically enriched compound of formula (III), or a salt thereof, 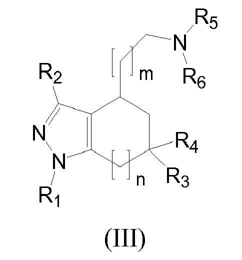 wherein R₁, R₂, R₃, R₄, n and m are as defined above; and
R₅ and R₆ are independently selected from the group consisting of hydrogen, substituted or unsubstituted alkyl, substituted or unsubstituted cycloalkyl, substituted or unsubstituted heterocyclyl, substituted or unsubstituted aryl, substituted **or** unsubstituted arylalkyl and substituted or unsubstituted heterocyclylalkyl or, R₅ and R₆ together form a substituted or unsubstituted heterocyclyl having 3 to 7 atoms in the ring;
which comprises reacting a mixture of C4-enantiomers of an ester of formula (I), as defined above, or salts thereof, with an esterase enzyme in the presence of a solvent, and
further comprises transforming the obtained enantiomerically enriched ester of formula (I) and/or the enantiomerically enriched acid of formula (II), or salts thereof, into the compound of formula (III), or a salt thereof.

Said compounds of formula (III) or salts thereof correspond to the pyrazole derivatives disclosed in WO 2006/021463 A1 which show chirality in the carbon 4.

Finally, a further aspect of the invention is a process or the synthesis of a compound of formula (IV), or a salt thereof, 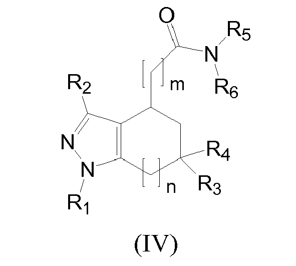 wherein R₁, R₂, R₃, R₄, R₅, R₆ n and m are as defined above,
comprising the transformation of an acid of formula (II) 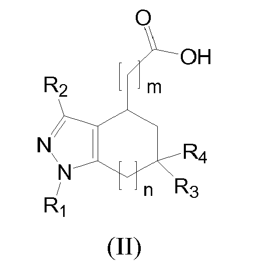 wherein R₁, R₂, R₃, R₄, n and m are as defined above,
into its corresponding acyl halide in the presence of an acyl halide promoter and its further condensation with an amine of formula HNR₅R₆ to provide an amide of formula (IV).

The inventors have found that the transformation of the acyl halide of an acid of formula (II) provides, in the presence of an amine of formula HNR₅R₆, the compounds of formula (IV), also intermediates towards the compounds of formula (III), in much higher yield.

Although the enzymatic resolution of other esters having a different structure is known in the prior art, it has been surprising to the inventors that such strategy could be used in the present situation. Further, it has been surprising that the enantiomers are obtained with good to excellent yields and enantiomeric excess. This discovery will allow scaling up the production of the pyrazole derivatives disclosed in WO 2006/021463 A1.

### DETAILED DESCRIPTION OF THE INVENTION

### Definitions

The following terms have the meaning indicated:

For the purposes of the present invention, when a mixture is "enantiomerically enriched" means that one enantiomer is obtained in excess of the other enantiomer. Therefore, an enantiomerically enriched mixture have an enantiomeric excess of over 0%, preferably over 20%, preferably over 40%, preferably over 70%, more preferably over 80%, more preferably over 90% or more preferably over 95% of one enantiomer in relation to the other enantiomer.

An "enantiomerically pure" compound is considered as a mixture of two enantiomers where said mixture is composed of over 95%, preferably over 98%, more preferably over 99%, or more preferably over 99.5% of one enantiomer.

The term "resolution" or "resolve" as used herein refers to a procedure through which the enantiomeric excess of a mixture of two enantiomers is increased. For example, the resolution of a racemic mixture refers to a procedure in which from an equimolar mixture of two enantiomers, a mixture wherein one of the enantiomers is present in a higher ratio with respect to the other one, is obtained. Similarly, it is possible to start from a mixture of two enantiomers wherein one of the enantiomers is present in a higher ratio than the other one, and as a result of the resolution process, the final ratio of the two enantiomers is different from the original ratio.

"Alkyl" refers to a straight or branched unsaturated hydrocarbon chain radical consisting of carbon and hydrogen atoms, having 1 to 12, preferably 1 to 8 carbon atoms, more preferably 1 to 4, and which is attached to the rest of the molecule by a single bond, e. g., methyl, ethyl, n-propyl, i-propyl, n-butyl, t-butyl, n-pentyl, etc.

"Alkenyl" refers to a straight or branched hydrocarbon chain radical consisting of carbon and hydrogen atoms, at least once saturated, having 1 to 12, preferably 1 to 8 carbon atoms, more preferably 1 to 4, and which is attached to the rest of the molecule by a single bond, e. g., vinyl, allyl, butenyl (e.g. 1-butenyl, 2-butenyl, 3-butenyl), pentenyl (e.g. 1-pentenyl, 2-pentenyl, 3-pentenyl, 4-pentenyl,), hexenyl (e.g. 1-hexenyl, 2-hexenyl, 3-hexenyl, 4-hexenyl, 5-hexenyl,), butadienyl, pentadienyl (e.g. 1,3-pentadienyl, 2,4-pentadienyl), hexadienyl (e.g. 1,3-hexadienyl, 1,4-hexadienyl, 1,5-hexadienyl, 2,4-hexadienyl, 2,5-hexadienyl), 2-ethylhexenyl (e.g. 2-ethylhex-1-enyl, 2-ethylhex-2-enyl, 2-ethylhex-3-enyl, 2-ethylhex-4-enyl, 2-ethylhex-5-enyl), 2-propyl-2-butenyl.

"Cycloalkyl" refers to a stable 3 to 10, preferably 5, 6 or 7 membered monocyclic or bicyclic unsaturated hydrocarbon radical, and which is attached to the rest of the molecule by a single bond, such as cyclohexyl or adamantyl.

"Aryl" refers to single or multiple ring radicals, having 6 to 10 carbon atoms, fused or not fused (bonded through one bond), including at least one aromatic ring radical. Typical aryl groups contain 1 or 2 separated or fused rings, such as phenyl, naphthyl, tetralin, indenyl, or phenanthryl radicals.

"Arylakyl" refers to an aryl group linked to the rest of the molecule through an alkyl group, such as benzyl and phenethyl.

"Heterocyclyl" refers to a stable 3-to 15 membered ring radical, aromatic or not, which consists of carbon atoms and from one to five heteroatoms selected from the group consisting of nitrogen, oxygen, and sulphur, preferably a 4-to 8-membered ring with one or more heteroatoms, more preferably a 5-or 6-membered ring with one or more heteroatoms. For the purposes of this invention, the heterocycle may be aromatic, saturated or partially saturated, and also a monocyclic, bicyclic or tricyclic ring system, which may include fused ring systems; and the nitrogen, carbon or sulphur atoms in the heterocyclyl radical may be optionally oxidised; the nitrogen atom may be optionally quaternized. Examples of such heterocycles include, but are not limited to, azepines, benzimidazole, benzothiazole, furan, isothiazole, imidazole, indole, piperidine, piperazine, purine, quinoline, thiadiazole, tetrahydrofuran, coumarine, morpholine; pyrrole, pyrazole, oxazole, isoxazole, triazole, imidazole, etc.

"Heterocyclylalkyl" refers to a heterocyclyl group linked to the rest of the molecule through an alkyl group.

When making reference to a 3 to 6 or 7 membered ring it is meant a cycloalkyl, or heterocyclyl group having respectively 3 to 6 or 7 ring members.

"Alkoxy" refers to a radical of the formula -OR^{a} where R^{a} is an alkyl radical as defined above, e. g., methoxy, ethoxy, propoxy, etc.

"Alkylthio" refers to a radical of the formula-SR^{a} where R^{a} is an alkyl radical as defined above, e. g., methylthio, ethylthio, propylthio, etc.

"Amino" refers to a radical of the formula-NH₂, -NHR^{a} or -NR^{a}R^{b}, optionally quaternized, wherein R^{a} and R^{b} are, independently, an alkyl radical as defined above, e.g., methylamino, ethylamino, dimethylamino, diethylamino, propylamino, etc.

"Halo" or "hal" refers to bromo, chloro, iodo or fluoro.

References herein to substituted groups in the compounds of the present invention refer to the specified moiety that may be substituted independently at one or more available positions by one or more suitable groups without changing the activity of the compound and which is compatible with the processes of the invention. Such moieties may be selected, for example, from the group consisting of halogen, such as fluoro, chloro, bromo and iodo; cyano; hydroxyl; nitro; azido; alkanoyl having 2 to 6 carbon atoms, such as acyl; alkyl, alkenyl or alkynyl groups having 1 to 4 carbon atoms; alkoxy groups having one or more oxygen linkages and having 1 to 4 carbon atoms; aryloxy groups having 6 to 10 carbon atoms, such as phenoxy; alkylthio groups having one or more, preferably 1 or 2, thioether linkages and from 1 to 6 carbon atoms; alkylsulfinyl groups having 1 to 10 carbon atoms, preferably 1 to 6 carbon atoms; alkylsulfonyl groups having 1 to 10 carbon atoms, preferably 1 to 6 carbon atoms; aminoalkyl groups such as groups having one or more N atoms, preferably 1 or 2, and from 1 to 12 carbon atoms, preferably 1 to 6 carbon atoms; aryl groups having 6 to 10 carbon atoms, preferably phenyl or naphthyl; and arylalkyl groups having 7 to 11 carbon atoms, such as benzyl.

The system employed herein to depict the stereochemistry of chiral centres in the compounds and intermediates of the invention is the *flying wedge* notation, wherein solid wedges represent single bonds extending toward the viewer and the broken wedge represent single bonds extending below the plane of the paper, the solid lines indicate bonds in the plane (as reviewed in Maehr H. J. Chem. Ed., 1985, 62, 114-120).

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

"Salts" for instance, pharmaceutically acceptable salts of compounds provided herein are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, non-aqueous media like ether, ethyl acetate, ethanol, isopropanol, acetone or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic amino acid salts.

### Enzymatic Resolution

As mentioned above, a first aspect of the present invention is directed to a process for the enantioselective hydrolysis of a mixture of C4-enantiomers of esters of formula (I). The reaction yields a mixture of esters of formula (I) (not hydrolyzed) and of acids of formula (II) (hydrolyzed), or salts thereof, both enantiomerically enriched with respect of carbon 4. The inventors have discovered that each enantiomeric compound is isolated in excellent enantiomeric excess and improved yield with respect to the racemic synthesis described in the art. It must be taken into account that the election of the esterase may determine which of the two enantiomers of the ester of formula (I) is hydrolysed. Thus, the process of the present invention may provide an ester of formula (Ia) and an acid of formula (IIb) 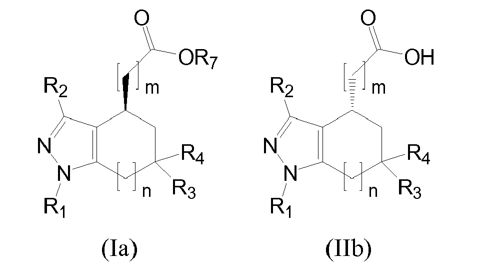 or an ester of formula (Ib) and an acid of formula (IIa).

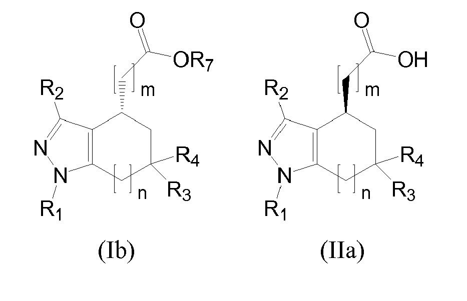

The enzymes suitable in principle for the process according to the invention are all esterases of nomenclature class 3.1, particularly preferred carboxylic ester hydrolases of nomenclature class 3.1.1, which react with carboxylic ester linkages. (nomenclature recommended by the Nomenclature Committee of the International Union of Biochemistry and Molecular Biology (NC-IUBMB)), which react with ester linkages. Examples of esterases of microbial origin which may be mentioned are esterases from fungi, yeasts or bacteria such as *Alcaligenes sp., Achromobacter sp., Aspergillus niger, Bacillus subtilis, Candida cylindracea, Candida lypolytica, Candida antarctica, Candida sp., Chromobacterium viscosum, Chromobacterium sp., Geotrichum candidum, Humicola lanuginosa, Mucor miehei, Penicillium camemberti, Penicillium roqueforti, Phycomyces nitens, Pseudomonas cepacia, Pseudomonas glumae, Pseudomonas fluorescens, Pseudomonas plantarii, Pseudomonas aeruginosa, Pseudomonas sp., Rhizopus arrhizus, Rhizopus delemar, Rhizopus japanicus, Rhizopus niveus, Rhizopus oryzae or Rhizopus sp.* Particularly preferred esterases are Lipases. Particularly preferred esterases are those from *Burkholderia* species such as *Burkholderia cepacia, Pseudomonas* species such as *Pseudomonas cepacia or Pseudomonas plantarii,* from *Candida* species such as *Candida cylindracea or Candida antarctica*, such as Novozym 435 or porcine pancreatic lipase. Very particularly preferred are Amano Lipase PS manufactured from a *Burkholderia cepacia* strain (supplied by Amano Pharmaceutical Co., Ltd, Japan) and Novozym SP523, SP524, SP525, SP526, SP539, SP435 (supplied by Novo, Denmark).

The esterases are employed in the reaction directly or as immobilizates on a wide variety of carriers. The amount of esterases to be added depends on the nature of the precursor, product, the ester and the activity of the esterase preparation. The optimal amount of esterase for the reaction can easily be determined by simple preliminary tests. The esterase /substrate ratio, calculated as molar ratio between esterase and substrate, depends on the esterase and is, as a rule, from 1:1000 to 1:50000000 or more, preferably 1:100000 to 1:5000000, which means that it is possible, for example to cleave 3 kg or more of a substrate with a molecular weight of about 100 to its enantiomers using 10 mg of an esterase.

The esterase reaction can be carried out without adding additional solvents or solvent mixtures only in the presence of the esters as solvent. It is advantageous to add other solvents or solvent mixtures to the reaction. All solvents inert in the reaction are suitable, that is they must not take part in the esterase reaction. Typical organic solvents include, but are not limited to alcohols, such as methanol or ethanol, or carbonyl compounds, such as ketones or aldehydes. The best results are obtained when a mixture of water and an organic solvent is used. Mixtures which are particularly suitable for the present invention are water/alcohol or water/ketone in any mixing ratio. According to a preferred embodiment, the solvent is a mixture of water with acetone or with isopropyl alcohol.

The course of the reaction can easily be followed by conventional methods, for example by gas chromatography. It is sensible to stop the reaction when 50% of the ester of formula (I) has reacted to obtain, in theory, maximum yield with maximum enantiomeric purity. The reaction may be stopped earlier or later, that is before or after 50% of the ester of formula (I) has reacted, to increase the enantiomeric purity. This usually takes place by removing the catalyst from the reaction, for example by filtering off the esterase.

The temperature of the reaction may be between -20°C and 75°C, preferably between 0°C and 40°C, more preferably between 15 and 35°C, more preferably room temperature. The reaction times depend on the substrate and the esterase used. Usually, a time of two hours or more are necessary, but the time can be less. According to a preferred embodiment, the reaction is between 1 hour and 7 days, more preferably between 5 hours and 6 days, even more preferably between 10 hours and 5 days. The average reaction time is between 12 hours and 96 hours.

The proportion of the esters of formula (Ia) and (Ib) in the initial reaction mixture is usually 1:1 (racemic mixture), but the process may be applied to mixtures having any proportion of esters of formula (Ia) and (Ib), in order to enantiomerically enrich the mixture with respect of one of them. Thus, the enantioselective hydrolysis of the present invention may result in the resolution of a racemic mixture of esters of formula (Ia) and (Ib) or in the enrichment of the mixture with respect of one of them.

Esters of formula (I), starting materials of the process of the invention, can be obtained as described in WO 2006/021463 A1. For example, see scheme I and related text on pages 16 and 17 of WO 2006/021463 A1.

Usually, it is preferred to obtain the compounds in enantiomeric pure form. However, the invention is useful as long as the compounds obtained are enantiomerically enriched. The process of the present invention may comprise further purification steps to increase optical purity or even enantiomerically pure compounds. Such purifications are common in the art, for example, recrystalization, chiral HPLC, or derivatization in diastereoisomers and further isolation. The skilled person may choose without difficulty the most appropriate purification procedure. It is also possible to repeat the process of the invention one or more times on the same mixture until the desired optical purity is obtained.

Therefore, the process according to the present invention may be also considered a preparation of an enantiomerically enriched ester of formula (I), or a salt thereof, an enantiomerically enriched acid of formula (II), or salts thereof, or a mixture of an enantiomerically enriched ester of formula (I) and an enantiomerically enriched acid of formula (II).

According to a preferred embodiment, R₂, R₃ and R₄ are hydrogen.

According to a preferred embodiment, R₇ is a C₁-₄ alkyl group.

According to a preferred embodiment, R₇ is a substituted C₅-C₁₂ cycloalkyl group, such as a cyclopentanol, cyclohexanol or menthol.

According to a preferred embodiment, R₁ is selected from the group consisting of substituted or unsubstituted C₆₋₁₀ aryl group, C₁₋₈ substituted or unsubstituted alkyl and heterocyclic.

According to a preferred embodiment, R₁ is a substituted or unsubstituted C₆₋₁₀ aryl group.

According to a preferred embodiment, ethyl (±)-2-(4,5,6,7-tetrahydro-1-phenyl-1H-indazole-4-yl)acetate reacts with an esterase enzyme.

### Synthesis of compounds of formula (III)

As mentioned above the enantiomerically enriched esters of formula (I) and the enantiomerically enriched acids of formula (II), are useful intermediates to obtain both enantiomers of the compounds of formula (III), which provide treatment for psychosis or pain.

### Sequence A

For example, following sequence A shown in scheme 1, an enantiomerically enriched acid of formula (II), for example and acid of formula (IIa), can be transformed into an enantiomerically enriched compound of formula (III), for example a compound of formula (IIIa)

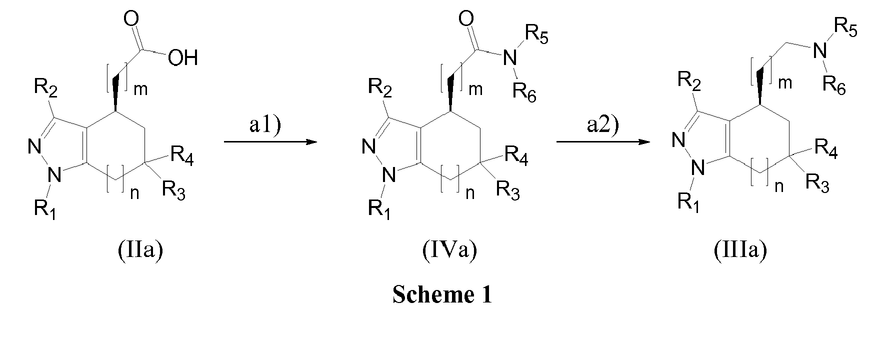

In a first step (step a1)) the method comprises reacting said enantiomerically enriched acid of formula (II), or a salt thereof, with an acid activating system in the presence of an amine of formula HNR₅R₆, wherein R₅ and R₆ are as defined above. Exemplary reagents and conditions are disclosed in WO 2006/021463 A1. See, for example, the transformation of compounds of formula XIII into compounds of formula XIV on pages 20-21 or the transformation of compounds of formula XV into compounds of formula XVI on pages 21-22 of WO 2006/021463 A1. Thus, alkyl chloroformates, such as ethyl chloroformate, isoprenyl chloroformate or isobutyl chloroformate, in the presence of a trialkylamine, such as triethylamine, ethyldiisopropylamine or methylmorpholine; or carbodiimides in the presence of 1-benzotriazole or N-hydroxysuccinimide; are suitable acid activating systems.

Further, the inventors have surprisingly found that by transforming an acid of formula (II), (IIa) or (IIb) into the corresponding acyl halides the yield of the reaction is significantly increased with respect to the methods disclosed in WO 2006/021463 A1. Thus, a further aspect of the invention is a process for the synthesis of a compound of formula (IV), comprising the transformation of an acid of formula (II) into its corresponding acyl halide in the presence of an acyl halide promoter and an amine of formula HNR₅R₆ 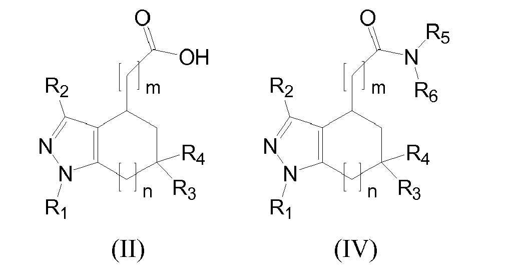 wherein
R₁, R₂, R₃, R₄, R₅, R₆, n and m are as defined above.

Typical acyl halide promoters are thionyl chloride, phosphorus pentachloride, phosphorus trichloride, phosphorus oxychloride, oxalyl chloride, oxalyl bromide, triphenylphosphine and carbon tetrachloride, hydrogen bromide in the presence of phosphoric or sulfuric acid, or hydrogen iodide in the presence of phosphoric or sulfuric acid, although other acyl halide promoters are known and may be used with similar results (reference is made to "Advanced Organic Chemistry: Reactions, Mechanism and Structure", 4th Ed., Wiley-Interscience, for example, see pages 437-438). According to a preferred embodiment, the acyl halide promoter is thionyl chloride. According to another preferred embodiment, the acyl halide promoter is oxalyl chloride.

The reaction usually takes place in an inert solvent, such as toluene, methylene chloride, tetrahydrofuran or N,N-dimethylformamide at an appropriate temperature which is typically between 0°C and the reflux temperature of the solvent.

Usually, the amine HNR₅R₆ is added once the acid is activated without isolation, but the acyl halide promoter and the amine may be added simultaneously. According to a preferred embodiment, the amine is morpholine.

In a second step (step a2)), sequence A comprises the reduction of the enantiomerically enriched amide of formula (IV), e.g. (IVa), resulting from step a1), into an enantiomerically enriched compound of formula (III), e.g. (IIIa), in the presence of a reducing agent. That is, reducing the amide group to amine in the presence of a reducing agent. Exemplary reducing agents may be selected from the group consisting of lithium aluminium hydride, di-iso-butyl aluminium hydride or triethyloxonium tetrafluoroborate and sodium borohydride. The reaction is carried out in inert solvents such as diethyl ether, tetrahydrofuran or toluene at a temperature between -78°C and reflux of the solvent. Further conditions may be selected by the skilled person, for example, by reference to March, J. "Advanced Organic Chemistry: Reactions, Mechanism and Structure", 4th Ed., Wiley-Interscience (for example, see pages 1206-1214, including table 19.5)

### Sequence B

Enantiomerically enriched compounds of formula (III), e.g. (IIIb), may be also obtained following the sequence B shown in scheme 2

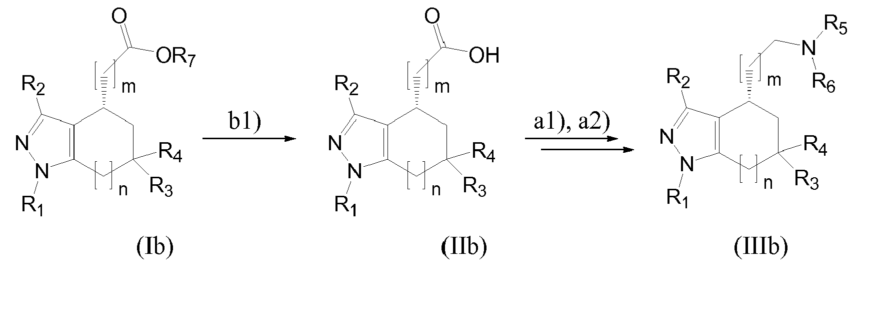

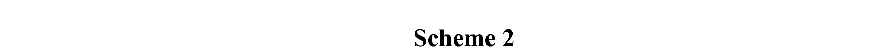

The first step (step b1)) comprises hydrolyzing the ester group of an enantiomerically enriched ester of formula (I), e.g. (Ib), to obtain the corresponding enantiomerically enriched acid of formula (II), or a salt thereof, e.g. (IIb). Such hydrolysis can be performed following methods known in the art, typically in a wet organic solvent or water, in the presence of a hydrolysis reagent, such as an inorganic hydroxide, preferably hydroxides from metals of groups I and II of the periodic table. According to a preferred embodiment, the hydrolysis reagent is selected from the group consisting of sodium hydroxide, potassium hydroxide and lithium hydroxide. According to a preferred embodiment, the solvent is a mixture of water and an alcohol, preferably methanol or ethanol, and the temperature is comprised between 0°C and the reflux temperature of the solvent, preferably at room temperature.

The enantiomerically enriched acid of formula (II), e.g. (IIb), may be further transformed into an enantiomerically enriched compound of formula (III), e.g. (IIIb), following sequence A (steps a1) and a2)) defined above.

### Sequence C

Enantiomerically enriched compounds of formula (III), e.g. (IIIb), may be further obtained following the sequence C shown in scheme 3

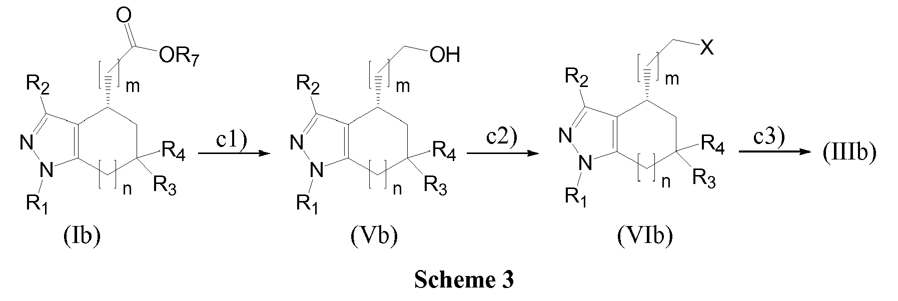

The first step (step c1)) comprises the reduction of the ester group of an enantiomerically enriched ester of formula (I), e.g. (Ib), in the presence of a reducing agent to obtain the corresponding enantiomerically enriched alcohol of formula (V), or a salt thereof, e.g. (Vb). That is, reducing the ester group to alcohol in the presence of a reducing agent. Exemplary reducing agents may be selected from the group consisting of lithium aluminium hydride, di-iso-butyl aluminium hydride or triethyloxonium tetrafluoroborate and sodium borohydride. The reaction is carried out in inert solvents such as diethyl ether, tetrahydrofuran, or toluene at a temperature between -78°C and reflux of the solvent. Further conditions may be selected by the skilled person, for example, by reference to March, J. "Advanced Organic Chemistry: Reactions, Mechanism and Structure", 4th Ed., Wiley-Interscience (for example, see pages 1206-1214, including table 19.5).

The next step (step c2)) comprises activating the alcohol of an enantiomerically enriched alcohol of formula (V) in the presence of a hydroxyl group activating reagent to obtain the enantiomerically enriched compound of formula (VI). X in the compounds of formula (VI) is a good leaving group. For the purposes of the present invention, the term "leaving group" has its commonly accepted meaning. For example, on page 205 of March, J. "Advanced Organic Chemistry: Reactions, Mechanism and Structure", 4th Ed., Wiley-Interscience, a leaving group is defined as the part of the molecule which becomes cleaved in the reaction. Suitable reagents to convert a hydroxyl group into a leaving group may be selected from the group consisting of thionyl chloride, phosphorus pentachloride, phosphorus trichloride, phosphorus (V) oxychloride, triphenylphosphine/carbon tetrachloride, hydrogen bromide, hydrogen iodide, methanesulfonyl chloride with triethyl amine and 4-methylbenzenesulfonyl chloride in pyridine. Thus, according to a preferred embodiment, X is selected from chlorine, bromine, iodide, methanesulfonate and 4-methylbenzenesulfonate. Further exemplary leaving groups may be found between pages 352 and 357 of March, J. "Advanced Organic Chemistry: Reactions, Mechanism and Structure", 4th Ed., Wiley-Interscience.

Finally, step c3) comprises reacting the compound resulting from c2) with an amine of formula HNR₅R₆, wherein R₅ and R₆ are as defined above. Thus, such reaction comprises the nucleophilic substitution by HNR₅R₆ of the group X of an enantiomerically enriched compound of formula (VI), e.g. (VIb), to provide an enantiomerically enriched compound of formula (III), e.g. (IIIb). Typical conditions involve an inert solvent and a temperature which depends on the solvent used, and on the reactivity of the amine and the leaving group. Exemplary conditions can be found in WO 2006/021463 A1 (e.g., transformation of compounds of formula IIa into Ia - pages 16 and 17; or Example 1, pages 26-27). Also, in March, J. "Advanced Organic Chemistry: Reactions, Mechanism and Structure", 4th Ed., Wiley-Interscience (pages 357-362) a discussion of the medium effects (e.g. solvent polarity) can be found.

### Sequence D

Enantiomerically enriched compounds of formula (III), e.g. (IIIb), may be also obtained following the sequence D shown in scheme 4

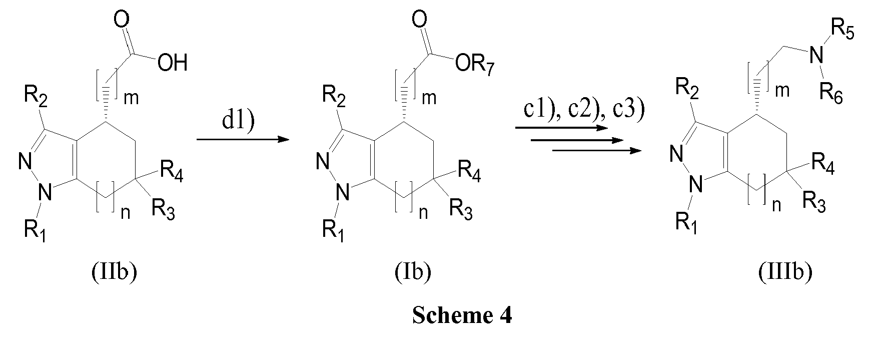

The first step (step d1)) comprises transforming an enantiomerically enriched acid of formula (II), e.g. (IIb), or a salt thereof, into an enantiomerically enriched ester of formula (I), e.g. (Ib). Such transformation can be performed following methods known in the art, typically in an organic solvent, in the presence of alkylating reagent, such as an alkyl halide, preferably alkyl iodides. According to a preferred embodiment, the solvent is a polar organic solvent, such as DMF, and the temperature is comprised between 0°C and the reflux temperature of the solvent. Conditions for esterification of carboxylic acids are well known in the art, and a number of conditions are available to the skilled person. For example, pages 393-396 and 398-400 of March, J. "Advanced Organic Chemistry: Reactions, Mechanism and Structure", 4th Ed., Wiley-Interscience describes some of the most common esterification processes. For example, esterification in acid media in the presence of an alcohol may be assisted by dehydrating agents such as DCC or DHU, or the reaction with diazo compounds.

The enantiomerically enriched ester of formula (I), e.g. (Ib), may be further transformed into an enantiomerically enriched compound of formula (III), e.g. (IIIb), following sequence C (steps c1), c2) and c3)) defined above.

Thus, sequence C and sequence D are complementary. The enantioselective hydrolysis of the invention provides an enantiomerically enriched acid of formula (II) and an enantiomerically enriched ester of formula (I), both having opposite spatial configuration at C4. Following sequence D, the enantiomerically enriched acid of formula (II) may provide access to one of the enantiomers of formula (III), while sequence C access to the opposite enantiomer of formula (III), in both cases with the same synthetic method. That is, from the same racemic starting material, both enantiomers of a compound of formula (III) can be prepared following the same sequence, and therefore using the same materials.

The following non-limiting examples describe the invention in detail. They are given only as further illustration of the invention, and they should not be taken as a definition of the limits of the invention.

### EXAMPLES

### Synthesis of (S)-4,5,6,7-tetrahydro-4-(2-(morpholin-4-yl)ethyl)-1-phenyl-1H-indazole hydrochloride and (R)-4,5,6,7-tetrahydro-4-(2-(morpholin-4-yl)ethyl)-1-phenyl-1H-indazole hydrochloride.

### Example 1: Synthesis of ethyl 2-(4,5,6,7-tetrahydro-1-phenyl-1H-indazol-4-yl)acetate.

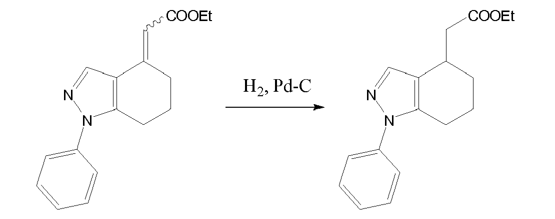

5% Pd-C (6.0 g) is added to a solution of a mixture of E/Z isomers of ethyl (1,5,6,7-tetrahydro-1-phenyl-4H-indazol-4-ylidene)acetate (22.0 g, 77.9 mmol) in EtOH (800 mL), and the resulting mixture is stirred under a hydrogen atmosphere (35 psi) in a Parr hydrogenator for 18 hours. The reaction mixture is purged with nitrogen, filtered through Celite and the solvent is evaporated under reduced pressure, obtaining ethyl 2-(4,5,6,7-tetrahydro-1-phenyl-1H-indazol-4-yl)acetate (22.0 g, 77.4 mmol, 99%, amber oil).

Alternatively, the hydrogenation can be carried out using AcOH or DMF as a solvent.
¹H NMR (300 MHz, CDCl₃): δ 1.31 (t, J= 7.2 Hz, 3H), 1.49 (m, 1H), 1.74 (m, 1H), 1.98 (m, 2H), 2.45 (dd, J= 15.4 Hz, J'= 8.2 Hz, 1H), 2.68 (m, 3H), 3.27 (m, 1H), 4.21 (q, J= 7.2 Hz, 2H), 7.32 (m, 1H), 7.41-7.52 (m, 5H).

### Example 2: Synthesis of (S)-2-(1-phenyl-4,5,6,7-tetrahydro-1H-indazol-4-yl)acetic acid and ethyl (R)-2-(1-phenyl-4,5,6,7-tetrahydro-1H-indazol-4-yl)acetate through resolution of ethyl (R,S)-2-(1-phenyl-4,5,6,7-tetrahydro-1H-indazol-4-yl)acetate.

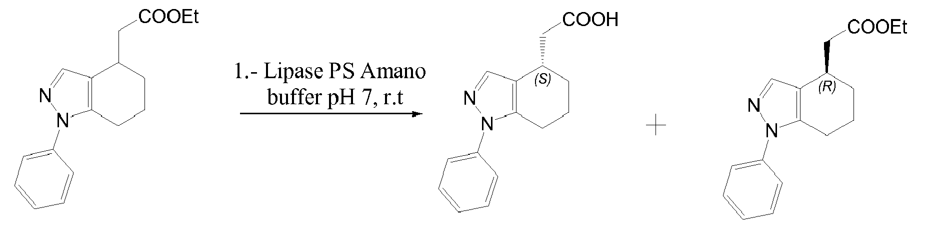

A mixture of ethyl (*R,S*)2-(4,5,6,7-tetrahydro-1-phenyl-1H-indazol-4-yl)acetate (28.0 g, 98.5 mmol), acetone (70 mL), phosphate buffer (125 mL, pH 7.0, 0.05 M) and water (125 mL) is treated with Amano Lipase PS (28 g, Amano Pharmaceutical Co,. Ltd) and the resulting mixture is maintained under intense stirring for 96 hours. 2N HCl (2 mL) is added to the suspension and it is left 20 minutes under stirring. Ethyl acetate is added and the solids are filtered to improve the phase separation. The organic phase is separated and washed 3 times with a solution of Na₂CO₃ and this aqueous solution is again washed with ethyl acetate and pooled with the previous organic phase. The aqueous solution is acidified with 2N HCI and the precipitate formed is filtered, washed with water and dried, obtaining (*S*)-2-(1-phenyl-4,5,6,7-tetrahydro-1H-indazol-4-yl)acetic acid (9.1 g, 35.50 mmol, 36%, white solid, m.p. 116-118°C, ee >99%, [α]_{D}²⁰ + 4.3 (c= 1, MeOH)).
¹H NMR (300 MHz, CDCl₃): δ 1.52 (m, 1H), 1.76 (m, 1H), 1.95 (m, 1H), 2.06 (m, 1H), 2.52 (dd, J= 15.8 Hz, J'= 8.2 Hz, 1H), 2.68-2.81 (m, 3H), 3.29 (m, 1H), 7.34 (m, 1H), 7.48 (m, 4H), 7.58 (s, 1H).

The organic phase is evaporated under reduced pressure, obtaining (*R*)-ethyl 2-(4,5,6,7-tetrahydro-1-phenyl-1H-indazol-4-yl)acetate (13.85 g, 48.70 mmol, 49.44 %, ee>90%). This ester (12.5 g) is again treated two more times with Amano Lipase PS (9 g) in the same conditions to improve the enantiomeric purity of the ester (12.5g), obtaining (*R*)-ethyl 2-(4,5,6,7-tetrahydro-1-phenyl-1H-indazol-4-yl)acetate (11.8 g, 41.5 mmol, oil, 42%, ee>99.6%, [α]_{D}²⁰ - 7.1 (c= 0.5, MeOH)).
¹H NMR (300 MHz, CDCl₃): δ 1.30 (t, J= 7.1 Hz, 3H), 1.49 (m, 1H), 1.74 (m, 1H), 1.88-2.09 (m, 2H), 2.45 (dd, J= 15.3 Hz, J'= 8.1 Hz, 1H), 2.66-2.75 (m, 3H), 3.27 (m, 1H), 4.21 (q, J= 7.1 Hz, 2H), 7.33 (m, 1H), 7.42-7.52 (m, 5H).

### Example 3: Resolution conditions for different compounds of formula (I).

Two sets of experiments where run using Lipase PS Amano (table 1) or Novozym 435 (Table 2), under different reaction conditions and with different starting materials. As shown in tables 1 and 2, the method of the invention is generally applicable to compounds of formula (I) under different conditions. Also the improvement in enantiomeric excess after several resolution cycles is shown.

**Table 1: Resolution with Lipase PS Amano**

| Racemic Ester (conditions) | Enantiomerically enriched acid | Enantiomerically enriched ester |
|---|---|---|
| 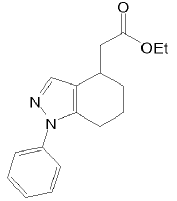 | 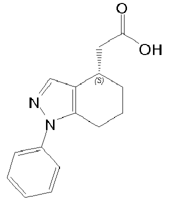 | 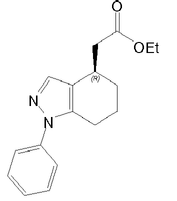 |
| Entry 1¹ (Conditions: same as example 2. | Enantiomeric excess after first cycle (ee >98,3%) [α]_{D}²⁰ +4,3 (c= 1, MeOH)). m.p.. 116-118°C white solid CHIRALPAK, OD-H, 5 µm, 0,46x25 cm. n-heptane/(IPA+0,5%TFA) 80/20 v/v φ= 0,7 mL/min | Enantiomeric excess after first cycle (ee >90%) Enantiomeric excess after third cycle (ee >99,6%) [α]_{D}²⁰ - 7,1 (c= 0.5, MeOH)). oil CHIRALPAK, OD-H, 5 µm, 0,46x25 cm n-heptane/IPA 90/10 v/v φ= 0,7 mL/min |
| 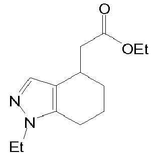 | 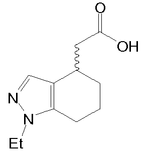 | 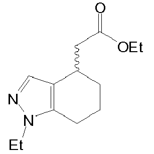 |
| Entry 2² (Conditions: **same as** example 2, but with a single resolution cycle of 36 hours at room temperature. | Enantiomeric excess after first cycle (ee >58%) Enantiomeric excess after second crystallization (ee >88%) [α]_{D}²⁰ -1,5 (c=1, MeOH) m.p. 182-184°C White solid CHIRALPAK, AD-H, 5 µm, 0,46x25 cm. n-heptane/IPA 80/20 v/v φ= 0,6 mL/min | Enantiomeric excess after first cycle (ee> 98,9%) [α]_{D}²⁰ +2,8 (c=1, MeOH) oil CHIRALPAK, AS-H, 5 □m, 0,46x25 cm. n-heptane/(EtOH+5%DEA) 98/2 v/v φ= 0,4 mL/min |
| 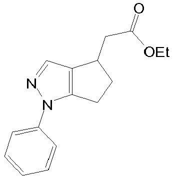 | 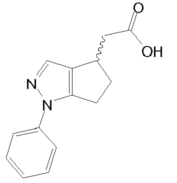 | 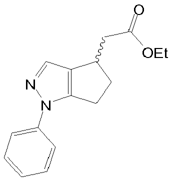 |
| Entry 3³ (Conditions: **same as** example 2, but with a single resolution cycle of 58 hours at room temperature. | Enantiomeric excess after first cycle (ee >94,8%) [α]_{D}²⁰+89,0 (c=1, MeOH) m.p. 130,0-130,2°C white solid CHIRALPAK, AD-H, 5 µm, 0,46x25 cm. n-heptane/IPA 90/10 v/v φ= 0,7 mL/min | Enantiomeric excess after first cycle (ee >96,2%) [α]_{D}²⁰ -61,8,0 (c=1, MeOH) oil CHIRALPAK, AD-H, 5 µm, 0,46x25 cm. n-heptane/IPA 90/10 v/v φ= 0,7 mL/min |

| | | |
|---|---|---|
| ¹ Retention times for esters (*S*) and (*R*) where tᵣ₁= 12,0 min and tᵣ₂= 12,8 min. Retention times for acids (*S*) and (*R*) where (*S*) y (*R*) son tᵣ₁= 8,2 min y tᵣ₂= 14,0 min. ² Retention times for enantiomers of the esters where tᵣ₁= 25,6 min y tᵣ₂= 27,5 min. Retention times for enantiomers of the acids where tᵣ₁= 13,8 min y tᵣ₂= 30,2 min. Absolute configuration of each enantiomer has not been determined. ³ Retention times for enantiomers of the esters where tᵣ₁= 15,0 min y tᵣ₂= 16,4 min. Retention times for enantiomers of the acids where tᵣ₁= 19,0 min y tᵣ₂= 31,12 min. Absolute configuration of each enantiomer has not been determined. | | |

In entry 1 the absolute configuration of the chiral centre was determined by comparison with the final product:

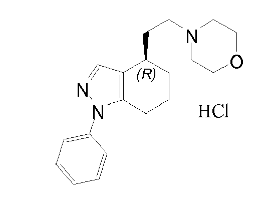

**Table 2: Resolution with Novozym 435**

| Racemic ester (conditions) | Enantiomerically enriched acid | Enantiomerically enriched ester |
|---|---|---|
| 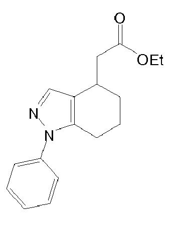 | 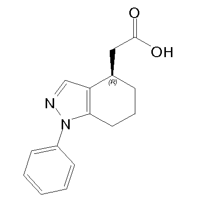 | 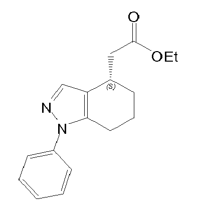 |
| Entry 1 (conditions: isopropylic ether /H₂O; ; room temperature; 14 hours) | Ratio of enantiomers (*R*: *S*) 73,6:26,4 CHIRALPAK, OD-H, 5 µm, 0,46x25 cm. n-heptane/ (IPA+0,5%TFA) 80/20 v/v φ= 0,7 mL/min. | Ratio of enantiomers (*R*: *S*) 29,5:70,5 CHIRALPAK, OD-H, 5 µm, 0,46x25 cm. n-heptane/IPA 90/10 v/v φ= 0,7 mL/min |
| Entry 2 (conditions: isopropylic ether /H₂O; ; 0°C; 2 hours) | Ratio of enantiomers (*R*: *S*) 86,4:13,6 CHIRALPAK, OD-H, 5 µm, 0,46x25 cm. n-heptane/ (IPA+0,5%TFA) 80/20 v/v φ= 0,7 mL/min | Ratio of enantiomers (*R*: *S*) 46,3:53,7 CHIRALPAK, OD-H, 5 µm, 0,46x25 cm. n-heptane/IPA 90/10 v/v φ= 0,7 mL/min |

### Example 4: Synthesis of (S)-4,5,6,7-tetrahydro-4-(2-(morpholin-4-yl)ethyl)-1-phenyl-1H-indazole hydrochloride according to sequence A.

### Step 1 (step a1)): Synthesis of (S)-4-(2-(1-phenyl-4,5,6,7-tetrahydro-1H-indazol-4-yl)acetyl) morpholine.

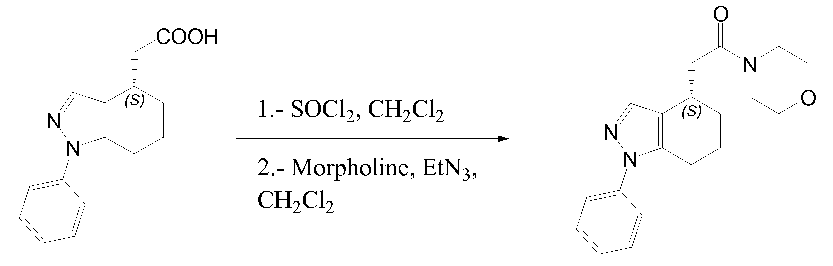

Thionyl chloride (30 mL) and a drop of DMF are added to a solution of (*S*)-2-(1-phenyl-4,5,6,7-tetrahydro-1H-indazol-4-yl)acetic acid (9.05 g, 35.3 mmol) in methylene chloride (50 mL). The solution is heated to boiling for 5 minutes and it is subsequently evaporated to dryness. The resulting residue is diluted in methylene chloride (40 mL) and is added drop wise on a mixture of triethylamine (10 mL) and morpholine (3.5 g, 40.2 mmol) in methylene chloride (60 mL) cooled in an ice bath and it is left under stirring for 30 minutes at room temperature. The solution is washed twice with water and the organic phase is separated, dried and evaporated to dryness, resulting in a crude product (11.8 g) which is purified by silica gel chromatography eluting with ethyl acetate, obtaining (*S*)-4-(2-(1-phenyl-4,5,6,7-tetrahydro-1H-indazol-4-yl)acetyl)morpholine (11.3 g, 34.7 mmol, 98%, amber oil which solidifies on standing, m.p. 111-113°C, [α]_{D}²⁰ + 21.0 (c= 0.65, MeOH)).
¹H NMR (400 MHz, CDCl₃): δ 1.46 (m, 1H), 1.78 (m, 1H), 1.93 (m, 1H), 2.08 (m, 1H), 2.45 (dd, J= 15.4 Hz, J'= 8.0 Hz, 1H), 2.66-2.76 (m, 3H), 3.38 (m, 1H), 3.49 (m, 2H), 3.63-3.76 (m, 6H), 7.33 (m, 1H), 7.47 (m, 4H), 7.51 (s, 1H).

### Step 2 (step a2)) Synthesis of (S)--4,5,6,7-tetrahydro-4-(2-(morpholin-4-yl)ethyl)-1-phenyl-1H-indazole.

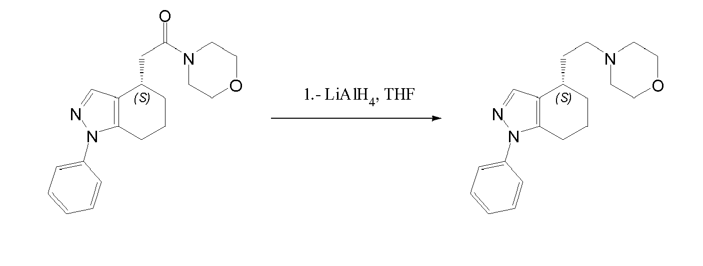

A solution of (*S*)-4-(2-(1-phenyl-4,5,6,7-tetrahydro-1H-indazol-4-yl)acetyl)morpholine (11.4 g, 35.0 mmol) in THF (40 mL) is added to a suspension of lithium aluminium hydride (5.6 g, 147.6 mmol) in THF (60 mL) cooled in an ice bath. The suspension is left under stirring at room temperature for 6 hours and it is then refluxed for 10 minutes. The suspension is cooled in an ice bath, slowly adding ice, and then 10% NaOH (1 mL) is added and it is left for 10 minutes under stirring. The solid formed is filtered and washed with ethyl acetate. The organic phase is dried and evaporated to dryness, obtaining (*S*)-4,5,6,7-tetrahydro-4-(2-(morpholin-4-yl)ethyl)-1-phenyl-1H-indazole, (10.4 g, 33.4 mmol, 95%, yellow oil, [α]_{D}²⁰ - 2.0 (c= 1, MeOH), [α]D²⁰ - 5.8 (c= 1, CDCl₃)).
¹H NMR (300 MHz, CDCl₃): δ 1.44 (m, 1H), 1.67 (m, 2H), 1.95 (m, 3H), 2.41-2.59 (m, 6H), 2.72 (m, 2H), 2.79 (m, 1H), 3.75 (t, J= 4.6 Hz, 4H), 7.32 (m, 1H), 7.47 (m, 4H), 7.53 (s, 1H).

### Step 3 (Salt Formation): Synthesis of (S)-4,5,6,7-tetrahydro-4-(2-(morpholin-4-yl)ethyl)-1-phenyl-1H-indazole hydrochloride.

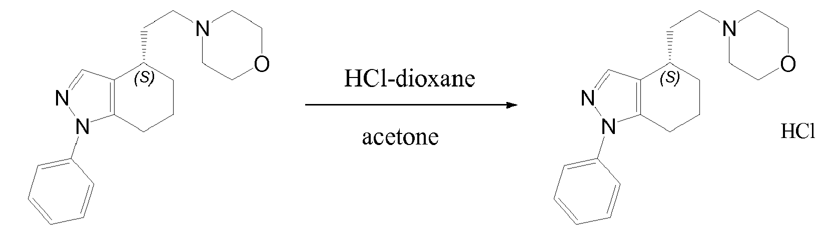

4.0 M HCI in dioxane (8.35 mL, 33.4 mmol) is added to a solution of (*S*)-4,5,6,7-tetrahydro-4-(2-(morpholin-4-yl)ethyl)-1-phenyl-1H-indazole (10.4 g, 33.4 mmol) in a mixture of acetone (100 mL) and ethanol (5 mL) maintained at room temperature and with intense stirring, adjusting the pH so that it is neutral or very slightly acid. The precipitate formed is filtered and washed with acetone, obtaining (*S*)-4,5,6,7-tetrahydro-4-(2-(morpholin-4-yl)ethyl)-1-phenyl-1H-indazole hydrochloride (9.94 g, 28.6 mmol, 86%, slightly yellow solid, m.p. 221-223°C, [α]_{D}²⁰ - 1.4 (c= 1, MeOH)).
¹H NMR (400 MHz, DMSO-*d₆*): δ 1.38 (m, 1H), 1.62 (m, 1H), 1.90 (m, 3H), 2.16 (m, 1H), 2.74 (m, 3H), 3.06 (m, 2H), 3.20 (m, 2H), 3.45 (m, 2H), 3.77-3.96 (m, 4H), 7.36 (m, 1H), 7.53 (m, 4H), 7.64 (s, 1H), 11.29 (m, 1H).

### Example 5: Synthesis of (S)-4-(2-(1-phenyl-4,5,6,7-tetrahydro-1H-indazol-4-yl)acetyl) morpholine starting from (S)-2-(1-phenyl-4,5,6,7-tetrahydro-1H-indazol-4-yl)acetic acid and oxalyl chloride.

### Step 1 (step a1)): Synthesis of (S)-4-(2-(1-phenyl-4,5,6,7-tetrahydro-1H-indazol-4-yl)acetyl) morpholine using oxalyl chloride.

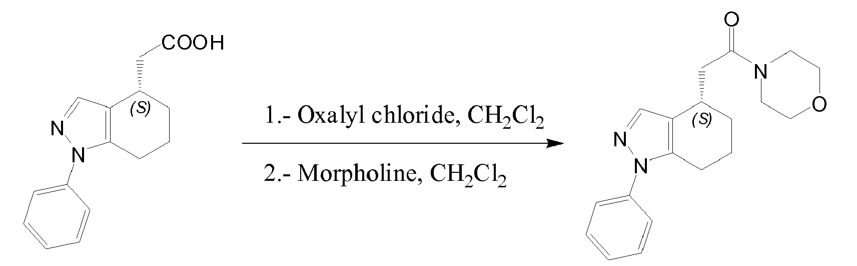

Oxalyl chloride (50 mg, 0.40 mmol) and a drop of DMF are added to a solution of (*S*)-2-(1-phenyl-4,5,6,7-tetrahydro-1H-indazol-4-yl)acetic acid (100 mg, 0.39 mmol) in methylene chloride (10 mL). The solution is heated to boiling for 20 minutes and it is subsequently evaporated to dryness. The resulting residue is diluted in methylene chloride (15 mL) and is added drop wise to a solution of morpholine (74.0 mg, 0.85 mmol) in methylene chloride (40 mL) cooled in an ice bath and it is left under stirring for 1 hour at room temperature. The solution is washed twice with water and the organic phase is separated, dried and evaporated to dryness, resulting in a crude which crystallized after treatment with diethyl ether, obtaining (*S*)-4-(2-(1-phenyl-4,5,6,7-tetrahydro-1H-indazol-4-yl)acetyl)morpholine (74.4 mg, 22.9 mmol, 59%, m.p. 112-113°C, [α]_{D}²⁰ + 24.5 (c= 1, MeOH)).

### Example 6: Synthesis of (R)-4,5,6,7-tetrahydro-4-(2-(morpholin-4-yl)ethyl)-1-phenyl-1H-indazole hydrochloride according to sequence B.

### Step 1 (step b1)) Synthesis of (R)-2-(1-phenyl-4,5,6,7-tetrahydro-1H-indazol-4-yl)acetic acid.

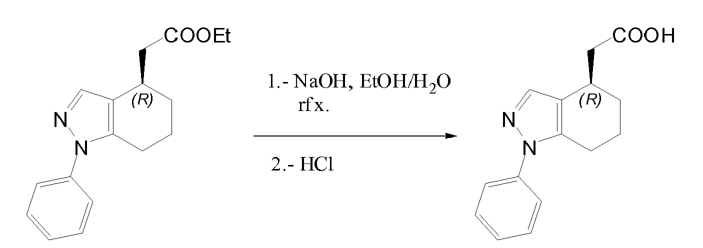

A mixture of (*R*)-ethyl 2-(4,5,6,7-tetrahydro-1-phenyl-1H-indazol-4-yl)acetate (23.0 g, 80.9 mmol), 1N NaOH (100 mL) and EtOH/H₂O (125 mL/25 mL) is refluxed for 8 hours. The ethanol is evaporated under reduced pressure and the mixture is acidified slightly with HCl, a paste which solidifies (it is extracted with ethyl acetate in the event that it does not solidify) being formed. The solid is filtered and dried, obtaining (*R*)-2-(1-phenyl-4,5,6,7-tetrahydro-1H-indazol-4-yl)acetic acid (20 g, 78.0 mmol, 96%, m.p. 110-112°C, [α]_{D}²⁰ 4.0 (c= 1, MeOH)).
¹H NMR (300 MHz, CDCl₃): δ 1.52 (m, 1H), 1.77 (m, 1H), 1.95 (m, 1H), 2.07 (m, 1H), 2.52 (dd, J= 15.7 Hz, J'= 8.1 Hz, 1H), 2.69-2.82 (m, 3H), 3.29 (m, 1H), 7.34 (m, 1H), 7.42-7.52 (m, 4H), 7.58 (s, 1H).

### Step 2 (step a1)): Synthesis of (R)-4-(2-(1-phenyl-4,5,6,7-tetrahydro-1H-indazol-4-yl)acetyl) morpholine.

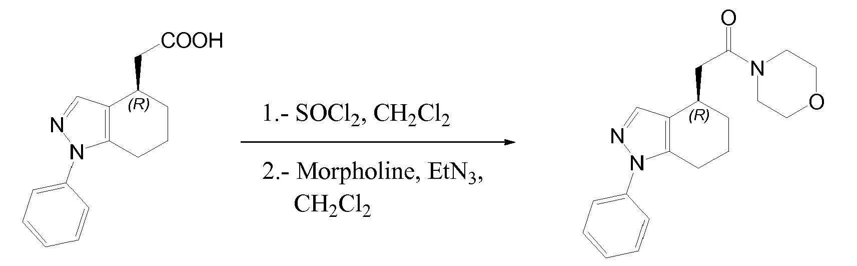

Thionyl chloride (40 mL) and a drop of DMF are added to a solution of (*R*)-2-(1-phenyl-4,5,6,7-tetrahydro-1H-indazol-4-yl)acetic acid (9.5 g, 37.1 mmol) in methylene chloride (100 mL). The solution is heated to boiling for 5 minutes and it is subsequently evaporated to dryness. The residue is diluted in methylene chloride (15 mL) and is added drop wise on a solution of triethylamine (16 ,5 mL) and morpholine (4.85 g, 55.7 mmol) in methylene chloride (100 mL) cooled in an ice bath and the resulting mixture is left under stirring for 30 minutes at room temperature. The solution is washed twice with water and the organic phase is dried and evaporated to dryness, obtaining (*R*)-4-(2-(1-phenyl-4,5,6,7-tetrahydro-1H-indazol-4-yl)acetyl)morpholine (11.25 g, 34.6 mmol, 93%, cream solid, m.p. 109-111°C, [α]_{D}²⁰ - 25.6 (c= 1, MeOH)).
¹H NMR (300 MHz, CDCl₃): δ 1.46 (m, 1H), 1.77 (m, 1H), 1.92 (m, 1H), 2.07 (m, 1H), 2.45 (dd, J= 15.4 Hz, J'= 7.8 Hz, 1H), 2.65-2.77 (m, 3H), 3.39 (m, 1H), 3.49 (m, 2H), 3.61-3.80 (m, 6H), 7.33 (m, 1H), 7.48 (m, 4H), 7.51 (s, 1H).

### Step 3 (step a2)): Synthesis of (R)-4,5,6,7-tetrahydro-4-(2-(morpholin-4-yl)ethyl)-1-phenyl-1H-indazole.

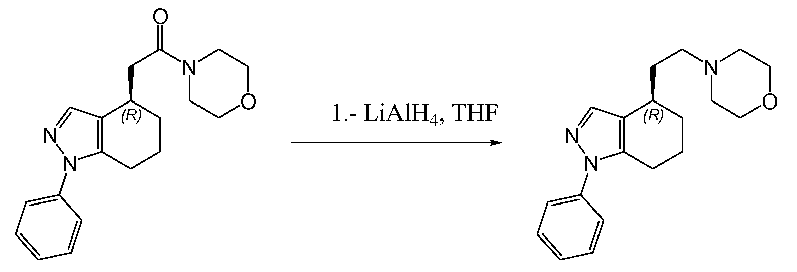

A solution of (*R*)-4-(2-(1-phenyl-4,5,6,7-tetrahydro-1H-indazol-4-yl)acetyl)morpholine (11.2 g, 34.6 mmol) in THF (25 mL) is added to a suspension of lithium aluminium hydride (5.0 g, 131.7 mmol) in THF (75 mL) cooled in an ice bath. The mixture is left under stirring at room temperature for 6 hours and it is then refluxed for 10 minutes. The suspension is cooled in an ice bath, ice is slowly added, and then 10% NaOH (1 mL) is added and it is left for 10 minutes under stirring. The solid is filtered and washed with ethyl acetate. The organic phase is dried and evaporated to dryness, obtaining (*R*)-4,5,6,7-tetrahydro-4-(2-(morpholin-4-yl)ethyl)-1-phenyl-1H-indazole, as an oil which solidifies on standing (10.2 g, 32.7 mmol, [α]_{D}²⁰ +2.5 (c= 1, MeOH), [α]_{D}²⁰ + 4.3 (c= 1, CDCl₃)).
¹H NMR (300 MHz, CDCl₃): δ 1.44 (m, 1H), 1.68 (m, 2H), 1.96 (m, 3H), 2.42-2.57 (m, 6H), 2.72 (m, 2H), 2.79 (m, 1H), 3.75 (m, 4H), 7.32 (m, 1H), 7.47 (m, 4H), 7.53 (s, 1H).

### Step 4 (salt formation): Synthesis of (R)-4,5,6,7-tetrahydro-4-(2-(morpholin-4-yl)ethyl)-1-phenyl-1H-indazole hydrochloride.

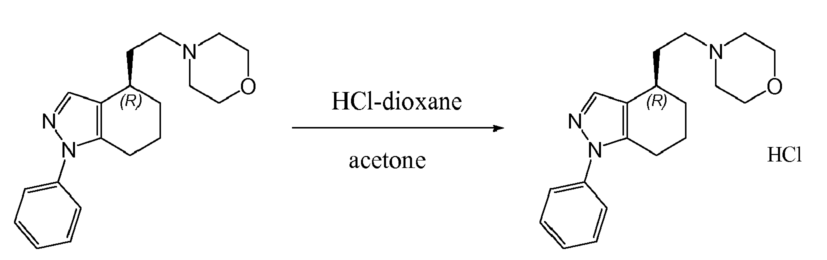

4.0 M HCl in dioxane (8.2 mL, 32.8 mmol) are added to a solution of (*R*)-4,5,6,7-tetrahydro-4-(2-(morpholin-4-yl)ethyl)-1-phenyl-1H-indazole (10.2 g, 32.7 mmol) in a mixture of acetone (80 mL) and ethanol (3 mL) maintained at room temperature and with intense stirring, adjusting the pH so that it is neutral or very slightly acid. The precipitate formed is filtered and washed with acetone, obtaining (*R*)-4,5,6,7-tetrahydro-4-(2-(morpholin-4-yl)ethyl)-1-phenyl-1H-indazole hydrochloride (10.2 g, 29.3 mmol, 90%, slightly yellow solid, m.p. 220-222°C, ee>99.9%, [α]_{D}²⁰ + 1.9 (c=1, MeOH)).
¹H NMR (400 MHz, DMSO-*d₆*): δ 1.38 (m, 1H), 1.62 (m, 1H), 1.90 (m, 3H), 2.16 (m, 1H), 2.64-2.82 (m, 3H), 3.06 (m, 2H), 3.19 (m, 2H), 3.45 (m, 2H), 3.84 (t, J_11,4, 2H), 3.94 (m, 2H), 7.36 (m, 1H), 7.47-7.55 (m, 4H), 7.63 (s, 1H), 11.33 (m, 1H).

### Example 7: Synthesis of (R)-4,5,6,7-tetrahydro-4-(2-(morpholin-4-yl)ethyl)-1-phenyl-1H-indazole hydrochloride according to sequence C.

### Step 1 (step c1)): Synthesis of (R)-2-(4,5,6,7-tetrahydro-1-phenyl-1H-indazol-4-yl)ethanol.

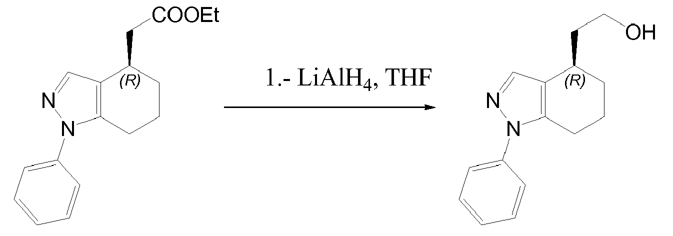

A solution of (*R*)-ethyl 2-(4,5,6,7-tetrahydro-1-phenyl-1H-indazol-4-yl)acetate (3.20 g, 11.25 mmol) in THF (20 mL) is added to a suspension of lithium aluminium hydride (2.30g, 60.60 mmol) in THF (80 mL) cooled at 0°C. The mixture is stirred for 5 hours and then refluxed for 45 minutes. The suspension is cooled, ice (approximately 5 g) and 10% NaOH (1.5 mL) are added. The white precipitate formed is filtered, and washed with ethyl acetate. The organic phase is dried and evaporated under reduced pressure, obtaining (*R*)-2-(4,5,6,7-tetrahydro-1-phenyl-1H-indazol-4-yl)ethanol (2.70 g, 11.14 mmol, 99%, oil, [α]_{D}²⁰ + 13.4 (c= 1, MeOH)).
¹H NMR (300 MHz, CDCl₃): δ 1.46 (m, 1H), 1.60-1.82 (m, 3H), 1.91-2.13 (m, 3H), 2.72 (m, 2H), 2.91 (m, 1H), 3.85 (t, J= 6.7 Hz, 2H), 7.32 (m, 1H), 7.41-7.53 (m, 4H), 7.54 (s, 1H).

### Step 2 (step c2): Synthesis of (R)-4-(2-chloroethyl)-4,5,6,7-tetrahydro-1-phenyl-1H-indazole.

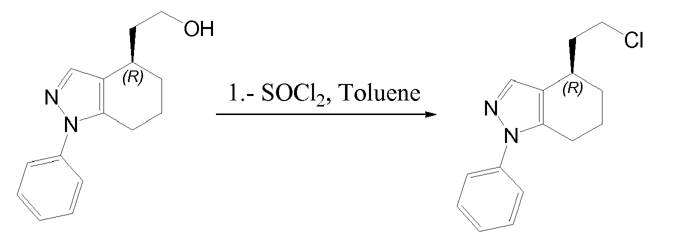

Thionyl chloride (6 mL) is slowly added to a solution of (*R*)-2-(4,5,6,7-tetrahydro-1-phenyl-1H-indazol-4-yl)ethanol (2.7 g, 11.14 mmol) in toluene (20 mL) and a drop of DMF at room temperature. The reaction mixture is heated at 60°C for 30 minutes. The solvent is eliminated under reduced pressure. The residue is diluted in ethyl acetate and washed twice with a saturated solution of NaHCO₃. The organic phase is dried and evaporated under reduced pressure, obtaining (*R*)-4-(2-chloroethyl)-4,5,6,7-tetrahydro-1-phenyl-1H-indazole (2.82 g, 10.81 mmol, 97%, oil, [α]₄₃₆²⁰ -11.1 (c= 1, MeOH)).
¹H NMR (300 MHz, CDCl₃): δ 1.45 (m, 1H), 1.74 (m, 1H), 1.94 (m, 3H), 2.20 (m, 1H), 2.73 (m, 2H), 3.01 (m, 1H), 3.72 (t, J= 6.7 Hz, 2H), 7.33 (m, 1H), 7.48 (m, 4H), 7.55 (s, 1H).

### Step 3 (step c3)): Synthesis of (R)-4,5,6,7-tetrahydro-4-(2-(morpholin-4-yl)ethyl)-1-phenyl-1H-indazole.

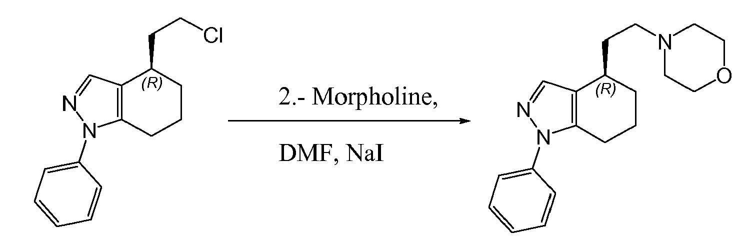

A mixture of (*R*)-4-(2-chloroethyl)-4,5,6,7-tetrahydro-1-phenyl-1H-indazole (2.6 g, 9.97 mmol), morpholine (2.65 g, 30.41 mmol) and a catalytic amount of KI in DMF (50 mL) is heated at 110°C for 5 hours. The solvent is eliminated under reduced pressure, the residue is diluted in ethyl ether, washed with a saturated solution of NaHCO₃, washed with water, and the organic phase is dried and evaporated under reduced pressure. The resulting crude product is purified by silica gel chromatography, obtaining (*R*)-4,5,6,7-tetrahydro-4-(2-(morpholin-4-yl)ethyl)-1-phenyl-1H-indazole (2.2 g, 7.06 mmol, 70%, oil, [α]_{D}²⁰ +4,4 (*c*= 1, MeOH)).
¹H NMR (300 MHz, CDCl₃): δ 1.44 (m, 1H), 1.70 (m, 2H), 1.96 (m, 3H), 2.39-2.60 (m, 6H), 2.72 (m, 2H), 2.79 (m, 1H), 3.75 (t, J= 4.7 Hz, 4H), 7.32 (m, 1H), 7.47 (m, 4H), 7.53 (s, 1H).

### Step 4 (Salt Formation): Synthesis of (R)-4,5,6,7-tetrahydro-4-(2-(morpholin-4-yl)ethyl)-1-phenyl-1H-indazole hydrochloride.

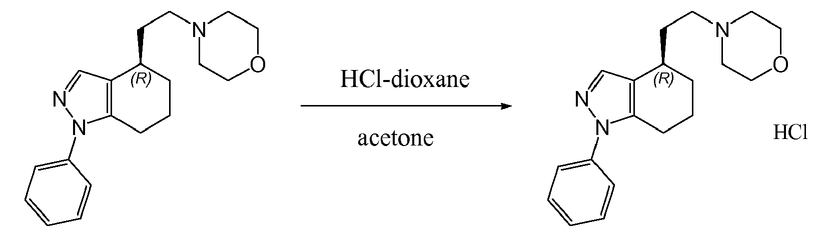

4.0 M HCl in dioxane (1.5 mL, 6.0 mmol) is added to a solution of (*R*)-4,5,6,7-tetrahydro-4-(2-(morpholin-4-yl)ethyl)-1-phenyl-1H-indazole (2.2 g, 7.06 mmol) in a mixture of acetone (60 mL) and ethanol (2 mL) maintained at room temperature and with intense stirring, adjusting the pH with 4.0 M HCl so that it is neutral or very slightly acid. The precipitate formed is filtered and washed with acetone, obtaining (*R*)-4,5,6,7-tetrahydro-4-(2-(morpholin-4-yl)ethyl)-1-phenyl-1H-indazole hydrochloride (2.0 g, 5.74 mmol, 81%, white solid, m.p. 218-221°C, ee>99,4%, [α]_{D}²⁰ + 2.0 (c= 1, MeOH)).
¹H RMN (300 MHz, CDCl₃): δ 1,39 (m, 1H), 1,63 (m, 1H), 1,89 (m, 3H), 2,14 (m, 1H), 2,74 (m, 3H), 3,06 (m, 2H), 3,22 (m, 2H), 3,46 (m, 2H), 3,79 (m, 2H), 3,96 (m, 2H), 7,36 (m, 1H), 7,46-7,57 (m, 4H), 7,63 (s, 1H), 10,88 (m, 1H).

### Example 8: Synthesis of (S)-4,5,6,7-tetrahydro-4-(2-(morpholin-4-yl)ethyl)-1-phenyl-1H-indazole hydrochloride according to sequence D.

### Step 1: methyl (S)-2-(4,5,6,7-tetrahydro-1-phenyl-1H-indazol-4-yl))acetate.

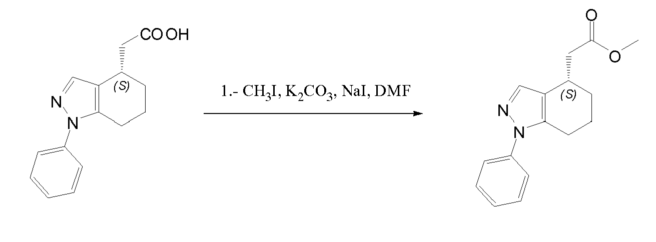

A mixture of (*S*)-2-(4,5,6,7-tetrahydro-1-phenyl-1H-indazol-4-yl))acetic acid (2,5 g, 9,75 mmol), methyl iodide (0,728 mL, 11,7 mmol), potassium carbonate (2,69 g, 19,5 mmol) and a catalytic amount ofNaI in DMF (50 mL) is heated to 100°C overnight. The mixture is diluted with water and extracted with ethyl acetate. The organic phase is separated, washed two times with water, dried and the solvent removed under reduced pressure, obtaining methyl (*S*)-2-(44,5,6,7-tetrahydro-1-phenyl-1H-indazol-4-yl)acetate (2,31 g, 8,55 mmol, 88%, dark oil, [α]_{D}²⁰ +10,2 (*c*= 1, MeOH)).
¹H NMR (400 MHz, CDCl₃): δ 1,48 (m, 1H), 1,76 (m, 1H), 1,94 (m, 1H), 2,02 (m, 1H), 2,47 (dd, J= 15,2 Hz, J'= 8,2 Hz, 1H), 2,72 (m, 3H), 3,28 (m, 1H), 3,75 (s, 3H), 7,33 (m, 1H), 7,43-7,51 (m, 5H).

### Step 2(step c1)): Synthesis of (S)-2-(4,5,6,7-tetrahydro-1-phenyl-1H-indazol-4-yl)ethanol.

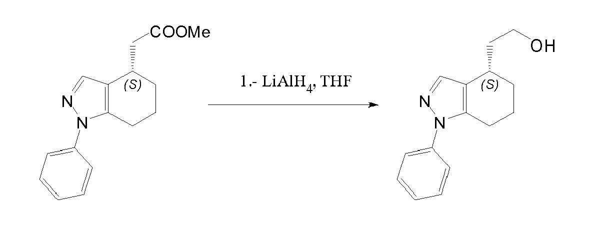

A solution of methyl (*S*)-2-(4,5,6,7-tetrahydro-1-phenyl-1H-indazol-4-yl)acetate (2,31 g, 8,55 mmol) en THF (20 mL)) is added to a suspension of lithium aluminum hydride (0,97 g, 25,6 mmol) in THF (80 mL)) cooled at 0°C. The mixture is stirred for 5 hours and then refluxed for 45 minutes. The suspension is cooled, ice (approximately 5 g) and 10% NaOH (1.5 mL) are added. The white precipitate formed is filtered, and washed with ethyl acetate. The organic phase is dried and evaporated under reduced pressure, obtaining (*S*)-2-(4,5,6,7-tetrahydro-1-phenyl-1H-indazol-4-yl)ethanol (1,79 g, 7,39 mmol, 86%, yellow oil, [α]_{D}²⁰ -15,3 (*c*= 1, MeOH)).
¹H NMR (400 MHz, CDCl₃): δ 1,47 (m, 1H), 1,58 (br s, 1H), 1,73 (m, 2H), 2,02 (m, 3H), 2,73 (m, 2H), 2,92 (m, 1H), 3,86 (m, 2H), 7,32 (m, 1H), 7,43-7,52 (m, 4H), 7,55 (s, 1H).

### Step 3 (step c2): Synthesis of (S)-4-(2-chloroethyl)-4,5,6,7-tetrahydro-1-phenyl-1H-indazole.

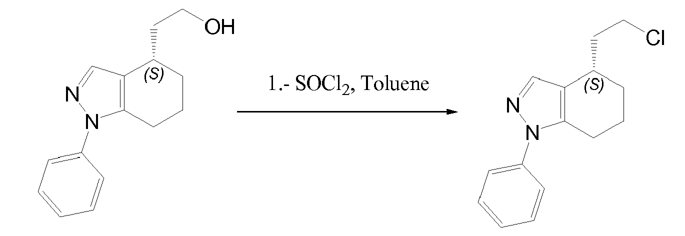

Thionyl chloride (4 mL) is slowly added to a solution of (*S*)-2-(4,5,6,7-tetrahydro-1-phenyl-1H-indazol-4-yl)ethanol (1.78 g, 7.38 mmol) in toluene (20 mL) and a drop of DMF at room temperature. The reaction mixture is heated at 60°C for 30 minutes. The solvent is eliminated under reduced pressure. The residue is diluted in ethyl acetate and washed twice with a saturated solution of NaHCO₃. The organic phase is dried and evaporated under reduced pressure, obtaining (*S*)-4-(2-chloroethyl)-4,5,6,7-tetrahydro-1-phenyl-1H-indazole (1.61 g, 6.17 mmol, 84%, oil). A sample (300 mg) is purified by chromatography over silica gel eluting with ethyl acetate-petroleum (1:1) ether (297 mg, [α]₄₃₆²⁰ +15.2 (c= 1, MeOH)).
¹H NMR (300 MHz, CDCl₃): δ 1,45 (m, 1H), 1,75 (m, 1H), 1,94 (m, 3H), 2,20 (m, 1H), 2,73 (m, 2H), 3,01 (m, 1H), 3,72 (t, J= 6,7 Hz, 2H), 7,33 (m, 1H), 7,48 (m, 4H), 7,55 (s, 1H).

### Step 4 (step c3)): Synthesis of (S)-4,5,6,7-tetrahydro-4-(2-(morpholin-4-yl)ethyl)-1-phenyl-1H-indazole.

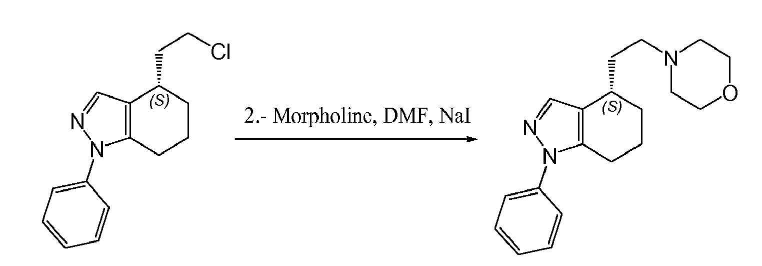

A mixture of (*S*)-4-(2-chloroethyl)-4,5,6,7-tetrahydro-1-phenyl-1H-indazole (200 mg, 0.77 mmol), morpholine (0198 mL, 2.28 mmol) and a catalytic amount of NaI in DMF (5 mL) is heated at 120°C overnight. The solvent is eliminated under reduced pressure, the residue is diluted in ethyl ether, washed twice with water, and the organic phase is dried and evaporated under reduced pressure. The resulting crude product (213 mg) is purified by treatment with oxalic acid dihydrate (86,1 mg, 0,68 mmol) obtaining the oxalate, which is then treated with diluted NaOH and extracted with CH₂Cl₂. The organic phase is dried and evaporated under reduced pressure obtaining (*S*)-4,5,6,7-tetrahydro-4-(2-(morpholin-4-yl)ethyl)-1-phenyl-1H-indazole as an oil which solidifies on standing (103 mg, 0,33 mmol, 43%, slightly orange oil, m.p. 76-78°C, [α]_{D}²⁰ -4,4 (*c*= 1, MeOH)).
¹H NMR (300 MHz, CDCl₃): δ 1,42 (m, 1H), 1,68 (m, 2H), 1,97 (m, 3H), 2,43-2,57 (m, 6H), 2,72 (m, 2H), 2,79 (m, 1H), 3,75 (m, 4H), 7,32 (m, 1H), 7,47 (m, 4H), 7,53 (s, 1H).

### Step 5 (Salt Formation): Synthesis of (S)-4,5,6,7-tetrahydro-4-(2-(morpholin-4-yl)ethyl)-1-phenyl-1H-indazole hydrochloride.

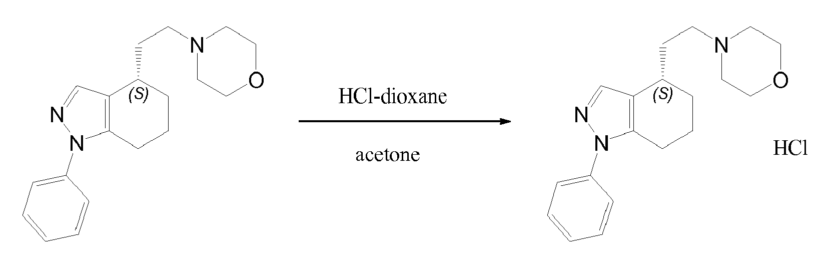

4.0 M HCI in dioxane (0,067 mL, 0,27 mmol) is added to a solution of (*S*)-4,5,6,7-tetrahydro-4-(2-(morpholin-4-yl)ethyl)-1-phenyl-1H-indazole (84,2 mg, 0,27 mmol) in a mixture of acetone (2 mL) maintained at room temperature, adjusting the pH with so that it is neutral or very slightly acid. The precipitate formed is filtered and washed with acetone, obtaining (*S*)-4,5,6,7-tetrahydro-4-(2-(morpholin-4-yl)ethyl)-1-phenyl-1H-indazole hydrochloride (50,0 mg, 0,14 mmol, 53%, slightly beige solid, m.p. 230-234°C, ee>97,1%, [α]_{D}²⁰ -1,3 (*c*= 1, MeOH)).
¹H NMR (300 MHz, DMSO-*d₆*): δ 1,39 (m, 1H), 1,63 (m, 1H), 1,89 (m, 3H), 2,12 (m, 1H), 2,73 (m, 3H), 3,07 (m, 2H), 3,22 (m, 2H), 3,47 (m, 2H), 3,77 (m, 2H), 3,97 (m, 2H), 7,36 (m, 1H), 7,52 (m, 4H), 7,63 (s, 1H), 10,67 (m, 1H).

## Claims

1. A process for the enantioselective hydrolysis of an ester of formula (I), which comprises reacting a mixture of C4-enantiomers of esters of formula (I), or salts thereof, with an esterase enzyme in the presence of a solvent 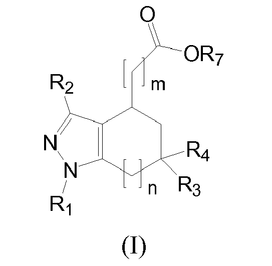 wherein
R₁ is selected from the group formed by hydrogen, substituted or unsubstituted alkyl, substituted or unsubstituted cycloalkyl, substituted or unsubstituted heterocyclyl, substituted or unsubstituted aryl, substituted or unsubstituted arylalkyl, and substituted or unsubstituted heterocyclylalkyl;
R₂ is selected from the group formed by hydrogen, substituted or unsubstituted alkyl, substituted or unsubstituted cycloalkyl, substituted or unsubstituted alkoxy, substituted or unsubstituted aryl, substituted or unsubstituted heterocyclyl, substituted or unsubstituted arylalkyl, and substituted or unsubstituted heterocyclylalkyl;
R₃ and R₄ are independently selected from the group formed by hydrogen, substituted or unsubstituted alkyl, substituted or unsubstituted cycloalkyl, substituted or unsubstituted heterocyclyl, substituted or unsubstituted aryl, substituted or unsubstituted arylalkyl and substituted or unsubstituted heterocyclylalkyl or, together, R₃ and R₄ form a 3 to 6 substituted or unsubstituted member ring;
R₇ is selected from the group formed by substituted or unsubstituted alkyl and substituted or unsubstituted cycloalkyl;
n is selected from 0, 1 and 2; and
m is selected from 0, 1, 2, 3 and 4.

2. A process for the preparation of an enantiomerically enriched ester of formula (I), or salts thereof, an enantiomerically enriched acid of formula (II), or salts thereof, or a mixture of an enantiomerically enriched ester of formula (I) and an enantiomerically enriched acid of formula (II) 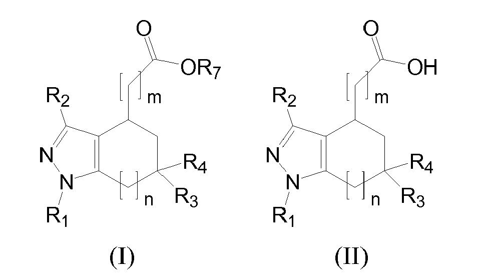 wherein R₁, R₂, R₃, R₄, R₇, n and m are as defined in claim 1;
comprising the step of reacting a mixture of C4-enantiomers of the ester of formula (I), or salts thereof, with an esterase enzyme in the presence of a solvent.

3. Process according to any of claims 1 or 2, wherein the esterase is a lipase selected from the group consisting of lipase Amano PS obtained from *Burkholderia cepacia* or Novozym 435 obtained from *Candida antarctica*.

4. Process according to any of the preceding claims, wherein R₂, R₃ and R₄ are hydrogen.

5. Process according to any of the preceding claims, wherein R₇ is a C₁₋₄ alkyl group.

6. Process according to any of the preceding claims, wherein R₁ is a substituted or unsubstituted C₆₋₁₀ aryl group.

7. Process according to any of the preceding claims wherein, ethyl (±)-2-(4,5,6,7-tetrahydro-1-phenyl-1H-indazole-4-yl)acetate reacts with an esterase enzyme.

8. Process for the synthesis of an enantiomerically enriched compound of formula (III), or a salt thereof, 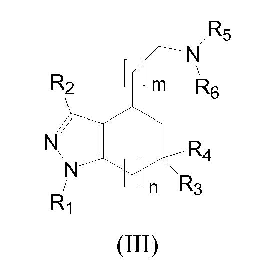 wherein R₁, R₂, R₃, R₄, n and m are as defined in claim 1; and
R₅ and R₆ are independently selected from the group consisting of hydrogen, substituted or unsubstituted alkyl, substituted or unsubstituted cycloalkyl, substituted or unsubstituted heterocyclyl, substituted or unsubstituted aryl, substituted or unsubstituted arylalkyl and substituted or unsubstituted heterocyclylalkyl or, R₅ and R₆ together form a substituted or unsubstituted heterocyclyl having 3 to 7 atoms in the ring;
which comprises reacting a mixture of C4-enantiomers of an ester of formula (I), as defined in any of claims 1 to 7, or salts thereof, with an esterase enzyme in the presence of a solvent, and
further comprises transforming the enantiomerically enriched ester of formula (I) and/or the enantiomerically enriched acid of formula (II), or salts thereof, obtained, into the compound of formula (III), or a salt thereof.

9. Process according to claim 8, wherein the transformation of said enantiomerically enriched ester of formula (I) and/or of said enantiomerically enriched acid of formula (II), or salts thereof, into the compound of formula (III), or a salt thereof, comprises at least one of sequence A, sequence B, sequence C or sequence D,
wherein
sequence A comprises
a1) reacting said enantiomerically enriched acid of formula (II), or a salt thereof, with an acid activating system in the presence of an amine of formula HNR₅R₆,
wherein R₅ and R₆ are as defined in claim 8; and
a2) reducing the resulting amide group to amine in the presence of a reducing agent;
sequence B comprises
b1) hydrolyzing the ester group of said enantiomerically enriched ester of formula (I) to obtain the corresponding enantiomerically enriched acid of formula (II), or a salt thereof; and
steps a1) and a2) defined above;
sequence C comprises
c1) reducing the ester group of said enantiomerically enriched ester of formula (I) in the presence of a reducing agent to obtain the corresponding alcohol;
c2) activating said alcohol in the presence of a hydroxyl group activating reagent; and
c3) reacting the compound resulting from the previous step with an amine of formula HNR₅R₆, wherein R₅ and R₆ are as defined in claim 8; and
sequence D comprises
d1) transforming said enantiomerically enriched acid of formula (II), or a salt thereof, into a enantiomerically enriched ester of formula (I); and
steps c1), c2) and c3) defined above.

10. The process according to claim 9, wherein the hydroxyl group activating reagent is selected from the group consisting of thionyl chloride, phosphorus pentachloride, phosphorus trichloride, phosphorus (V) oxychloride, hydrogen bromide, hydrogen iodide, methanesulfonyl chloride with triethyl amine and 4-methylbenzenesulfonyl chloride in pyridine.

11. The process according to any of claims 9 and 10, wherein the reducing agent is selected from the group consisting of lithium aluminium hydride, di-iso-butyl aluminium hydride or triethyloxonium tetrafluoroborate and sodium borohydride.

12. The process according to claim 9, wherein the enantiomerically enriched acid of formula (II), or a salt thereof, is transformed into an acyl halide.

13. Process for the synthesis of a compound of formula (IV), or a salt thereof, 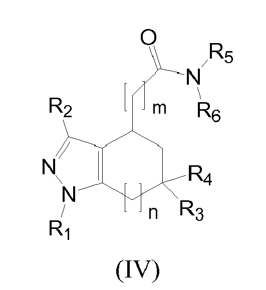 wherein
R₁, R₂, R₃, R₄, n and m are as defined in any of claims 1 to 7, and
R₅ and R₆ are independently selected from the group consisting of hydrogen, substituted or unsubstituted alkyl, substituted or unsubstituted cycloalkyl, substituted or unsubstituted heterocyclyl, substituted or unsubstituted aryl, substituted or unsubstituted arylalkyl and substituted or unsubstituted heterocyclylalkyl or, R₅ and R₆ together form a substituted or unsubstituted heterocyclyl having 3 to 7 atoms in the ring;
comprising the transformation of an acid of formula (II) 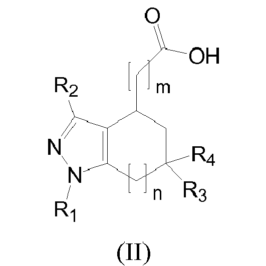 wherein R₁, R₂, R₃, R₄, n and m are as defined in any of claims 1 to 7,
into its corresponding acyl halide in the presence of an acyl halide promoter and an amine of formula HNR₅R₆.

14. Process according to claim 13, wherein said acyl halide promoter is oxalyl chloride or SOCl₂.
